# FASCICULE DE BREVET EUROPEEN

(11) **EP 0 885 872 B1**
(45) Date de publication et mention de la délivrance du brevet: **21.11.2001**
(21) Numéro de dépôt: 98401244.3
(22) Date de dépôt: 26.05.1998
(51) Int. Cl.: C07C 51/41, C07C 57/32, B01J 19/24

(54) **Procédé continu de fabrication de solutions aqueuses de sels alcalins d'acides arylacétiques.**
Kontinuierliches Verfahren zur Herstellung von wässrigen Lösungen von Alkalisalzen von Arylessigsäuren
Continuous process for the preparation of aqueous solutions of alkaline salts of arylcetic acids

(30) Priorité: 16.06.1997 FR 9707440
(43) Date de publication de la demande: 23.12.1998
(73) Titulaire: Atofina, 92800 Puteaux (FR)
(72) Inventeur: Rupin, Christophe, 69310 Pierre-Benite (FR); Gendarme, Joan-Philippe, 64230 Lescar (FR); Corbiere, Philippe, 95470 Survilliers (FR)

(56) Documents cités:
- FR-A- 2 004 194
- US-A- 2 817 681
- US-A- 4 031 243

## Description

L'invention concerne un procédé continu de fabrication de solutions aqueuses de sels alcalins d'acides arylacétiques.

Les sels alcalins des acides arylacétiques sont des matières premières importantes de l'industrie chimique. Ils sont très largement utilisés pour la synthèse de divers produits pharmaceutiques. Ainsi, les phénylacétates de sodium ou de potassium sont des intermédiaires de synthèse pour la préparation de la pénicilline G.

Egalement, ces sels alcalins des acides arylacétiques peuvent être des précurseurs pour la synthèse des acides arylacétiques et de leurs esters utilisés notamment pour la préparation de produits pharmaceutiques, de colorants et parfums.

Les méthodes couramment décrites pour obtenir les sels alcalins des acides arylacétiques consistent à réaliser l'hydrolyse alcaline des arylacétonitriles. Cette réaction d'hydrolyse alcaline est une réaction lente nécessitant des moyens d'agitation efficaces et un chauffage long lequel, compte tenu de l'instabilité des arylacétonitriles à la chaleur, est de nature à conduire à la formation de sous-produits abaissant le rendement et entraînant des opérations ultérieures de purification coûteuses, rédhibitoires pour un procédé industriel.

Les solutions aqueuses brutes alcalines telles qu'obtenues précédemment, c'est-à-dire non purifiées, peuvent être acidifiées pour conduire aux acides arylacétiques qui doivent être alors purifiées par distillation ou cristallisation.

Afin de raccourcir la période d'induction de la réaction d'hydrolyse alcaline, on préconise dans le brevet US 2,817,681 d'utiliser une quantité pondérale mineure (de préférence 5 % à 10 % par rapport à l'arylacétonitrile mis en oeuvre) de l'acide arylacétique (ou de son sel) correspondant à l'arylacétonitrile à hydrolyser.

Ainsi, l'addition de 5,4 % en poids d'acide méthoxy-3-éthoxy-4-phénylacétique par rapport au méthoxy-3-éthoxy-4 phénylacétonitrile. permet de réduire la durée de réaction d'environ 2 heures.

Cependant, on constate qu'il est nécessaire de purifier et de décolorer la solution acidifiée et que le rendement en acide méthoxy-3-éthoxy-4 phénylacétique passe seulement de 75 % à 80 % (sans addition mineur d'acide arylacétique lors de la réaction d'hydrolyse alcaline) à 91 % (avec addition).

Il est connu également que l'on peut préparer les acides arylacétiques par hydrolyse acide directe des arylacétonitriles. L'acide sulfurique est généralement utilisé, mais son utilisation présente de nombreux inconvénients. On note une mauvaise sélectivité du fait de la présence de sous-produits résultant notamment de la sulfonation des noyaux aromatiques. En conséquence, les rendements sont faibles et en outre il y a des effluents tels que le sulfate d'ammonium difficilement valorisable sans traitement ultérieur coûteux.

Les solutions aqueuses de sels alcalin desdits acides arylacétiques - obtenues par hydrolyse acide des arylacétonitriles - peuvent être obtenues par neutralisation desdits acides arylacétiques. Mais cette façon d'opérer est coûteuse compte tenu que la préparation desdits acides arylacétiques par hydrolyse acide des arylacétonitriles est une opération peu sélective et également onéreuse.

La demanderesse a trouvé un procédé continu, simple permettant de conduire en des temps très courts à des solutions aqueuses de sels alcalins des acides arylacétiques par hydrolyse alcaline des arylacétonitriles ne présentant pas tous ces inconvénients et, qui plus est, permet d'obtenir des acides arylacétiques purs par simple acidification desdites solutions.

L'invention concerne donc un procédé continu de préparation d'une solution aqueuse d'un sel alcalin d'acide arylacétique de formule : 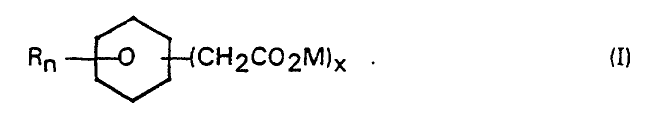 dans laquelle R représente un radical hydrocarboné aliphatique, linéaire ou ramifié ayant un nombre de carbone allant de 1 à 10, un atome d'halogène tel que chloré ou brome, un groupe alcoxy linéaire ou ramifié ayant un nombre de carbone allant de 1 à 6, un groupe phénoxy ; M représente un métal alcalin tel que Na ou K, n est un nombre, allant de O à 5, et x est un nombre allant de 1 à 2 avec n + x inférieur ou égal à 6 ; par hydrolyse alcaline d'un arylacétonitrile de formule : 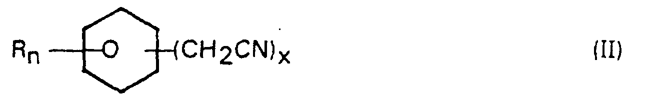 R, n et x ayant les mêmes significations que dans la formule (I) selon la réaction 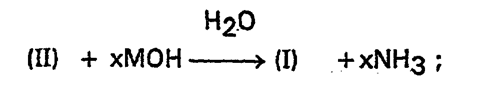 ledit procédé étant caractérisé en ce qu'il consiste essentiellement :
a) à mettre en contact dans une zone de mélange un arylacétonitrile (II) avec une solution aqueuse d'un hydroxyde alcalin MOH selon un rapport molaire MOH / (II) au moins égal à x et, de préférence, compris entre 1,05 x et 1,20 x,
b) à introduire le mélange obtenu en a) dans une zone de réaction sous une pression supérieure ou égale à la pression atmosphérique et, de préférence, comprise, entre 2 bars absolus et 12 bars absolus, et à maintenir ledit mélange dans ladite zone réactionnelle pendant une durée au plus égale à 60 minutes et, de préférence, comprise entre 5 minutes et 30 minutes, à une température comprise entre 100°C et 180°C et, de préférence entre 130°C et 160°C ;
c) à transférer le milieu obtenu en b) dans une zone de séparation,
d) à transférer le milieu obtenu en c) dans une zone de stripping,
e) à mettre en contact la solution obtenue en d) avec un agent neutralisant qui est un acide arylacétique de formule 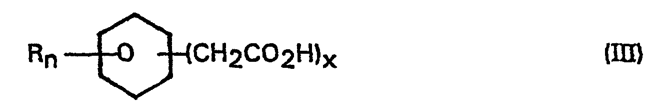 dans une zone de mélange, puis,
f) à récupérer une solution aqueuse d'arylacétate alcalin pure qui peut être éventuellement diluée avec de l'eau.

Selon la présente invention, la solution aqueuse d'hydroxyde alcalin MOH et l'arylacétonitrile (II) peuvent être préalablement préchauffés à une température au moins égale à 50°C et, de préférence à une température comprise entre 100°C et 130°C.

De préférence, on utilisera des températures de préchauffage des réactifs identiques, mais on ne sortirait pas du cadre de l'invention si on utilisait des températures de préchauffage des réactifs différentes.

Selon la présente invention, la technique de mélange utilisée dans l'étape a) doit être adaptée pour réaliser un mélange intime des réactifs.Le temps de contact entre les réactifs est, au plus égal à 10 minutes, et, de préférence va de 0,01 seconde à 6 minutes.

Selon la présente invention, la concentration pondérale en MOH des solutions aqueuses d'hydroxyde alcalin peut varier dans une large mesure. Elle est au moins égale à 10 % et, de préférence, comprise entre 20 % et 60 %.

D'une façon générale, on utilisera des solutions commerciales courantes.

Selon la présente invention, le milieu réactionnel obtenu dans l'étape b), qui se présente sous forme d'une solution aqueuse alcaline comprenant le sel alcalin d'un acide arylacétique et de l'ammoniac formé lors de la réaction, est introduit dans une zone de séparation dans laquelle la majeure partie de l'ammoniac est éliminée. Dans cette zone de séparation, la température et la pression sont, de préférence, au plus égales à celles de l'étape b).

L'ammoniac récupéré peut être avantageusement valorisée sous forme de solutions aqueuses.

La solutions aqueuse alcaline du sel alcalin d'acide arylacétique quasiment débarassée de son ammoniac est introduite dans une zone de stripping dans laquelle on élimine l'ammoniac résiduel.

La solution est strippée avantageusement avec de la vapeur d'eau surchauffée, de l'air ou un gaz inerte tel que l'azote.

La solution sortant de la zone de stripping de l'étape d) est mis en contact dans une zone de mélange avec une quantité suffisante d'un acide arylacétique de formule : 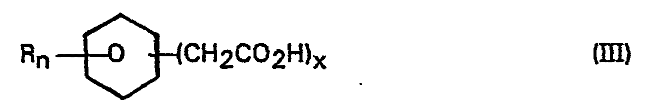 pour neutraliser l'éventuelle quantité d'hydroxyde alcalin non consommée.

Ensuite, on récupère une solution aqueuse de sel alcalin d'acide arylacétique qui peut être éventuellement diluée afin d'obtenir une solution d'arylacétate alcalin ayant une concentration pondérale en arylacétate alcalin adaptée à son utilisation ultérieure et pour obtenir une solution de sel alcalin d'acide arylacétique totalement soluble à température ambiante.

Ce procédé s'applique tout particulièrement à la préparation des phénylacétates de sodium ou de potassium, à partir de phénylacétonitrile.

L'invention concerne également une installation pour la fabrication de solutions aqueuses de sels alcalins des acides arylacétiques.

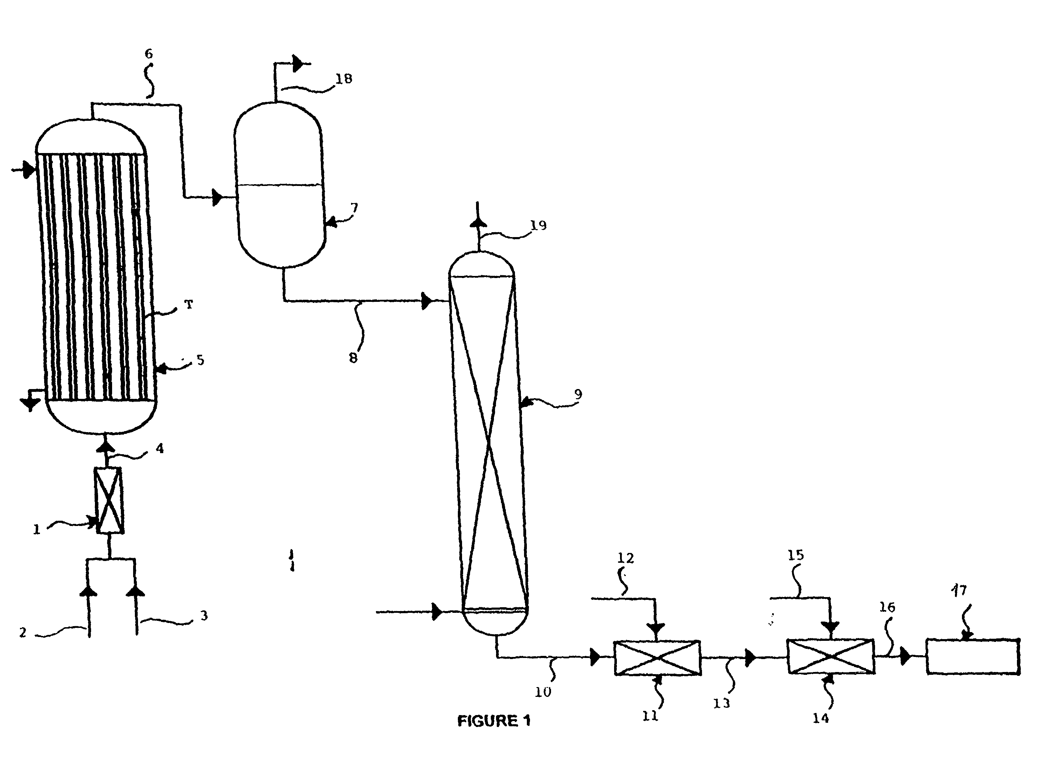

Cette installation représentée schématiquement sur la figure 1 comprend une enceinte (1) contenant des moyens de mélange munie d'une alimentation en arylacétonitrile (2) et d'une alimentation en solution aqueuse d'hydroxyde alcalin (3), une conduite (4) d'admission du mélange dans un réacteur (5) comprenant au moins un tube cylindrique (T) vertical vide, une conduite (6) d'admission de la solution aqueuse contenant un sel alcalin d'acide arylacétique et de l'ammoniac dans une enceinte (7) contenant des moyens de séparation gaz/liquide, une conduite (8) d'admission de la solution aqueuse dégazée dans une colonne de stripping (9), une conduite (10) d'admission de la solution aqueuse strippé dans une enceinte (11) contenant des moyens de mélange munie d'une alimentation en agent neutralisant (12), d'une conduite (13) d'admission dans une enceinte (14) contenant des moyens de mélange munie d'une alimentation (15) en eau, une conduite (16) d'admission de la solution aqueuse du sel alcalin d'acide arylacétique dans une zone de stockage (17) et des évents (18) et (19).

Selon la présente invention, les moyens de mélange contenus dans l'enceinte (1) peuvent être constitués par tout dispositif permettant d'obtenir un contact intime des réactifs.

Selon la présente invention, on utilisera tout particulièrement des mélangeurs statiques. Ces mélangeurs peuvent comprendre un ou plusieurs éléments mélangeurs identiques ou différents. Ces éléments mélangeurs peuvent comprendre des lamelles plissées ou un réseau de lames croisées, emboîtées les unes dans les autres.

Les réactifs sont introduits dans (1) par les alimentations (2) et (3). Ces réactifs peuvent être préchauffés par des moyens non représentés sur la figure 1. Le mélange sortant de (1) est transféré par la conduite (4) dans le réacteur (5) de préférence à sa partie inférieure.

On ne sortirait pas du cadre de l'invention si le ou les tube(s) contenait(aient) un garnissage vrac tel que anneaux Raschig, anneaux Pall, des billes, un garnissage ordonné.

Le nombre de tubes peut varier dans une large mesure. Il est proportionnel à la productivité des solutions aqueuses de sel alcalin d'acide arylacétique souhaitée.

Le réacteur (5) est muni de moyens de chauffage non représentés sur la figure 1, tels que fluide caloporteur, vapeur haute pression. Dans l'éventualité où l'on utilise de la vapeur d'eau, on ne sortirait pas du cadre de l'invention si cette vapeur d'eau était injectée avec le mélange des réactifs à l'intérieur du ou des tubes.

Afin d'assurer un chauffage initial homogène des tubes (T), on peut remplir préalablement lesdits tubes avec une solution aqueuse d'hydroxyde alcalin ou avec une solution aqueuse d'un sel alcalin d'acide arylacétique ou bien encore avec de l'eau.

La solution aqueuse, constituée essentiellement d'ammoniac et du sel alcalin de l'acide arylacétique, sortant du réacteur (5) est véhiculée par une conduite (6) dans une zone de séparation gaz/liquide constitué par un séparateur (7) qui peut être un bac vide de grande dimension dans lequel le gaz ammoniac se sépare de la solution aqueuse notamment par détente.

L'ammoniac gazeux est évacué dudit séparateur par l'évent (18). La solution aqueuse est transférée vers des moyens de stripping par une conduite (8). Selon la présente invention, les moyens de stripping peuvent être constitués par une colonne de stripping (9) contenant un garnissage vrac constitué notamment d'anneaux Raschig, d'anneaux Pall, de garnissage ordonné et à la base de laquelle on injecte un courant de vapeur d'eau, d'air ou d'un gaz inerte. En tête de ladite colonne, on élimine par l'évent (19) des gaz constitués majoritairement par de la vapeur d'eau, d'air ou de gaz inerte et de l'ammoniac résiduel. En bas de ladite colonne sort une solution aqueuse alcaline de sel alcalin d'acide arylacétique laquelle est véhiculée par une conduite (10) vers une première zone de mélange (11) constituée de préférence par des mélangeurs statiques tels que mentionnés précédemment. Ces mélangeurs peuvent être de type identique ou différent de ceux utilisés dans (1).

Cette zone de mélange (11) est alimentée en agent susceptible de neutraliser l'éventuel excès d'alcalinité par la conduite d'alimentation (12).

Selon la présente invention on utilisera de préférence comme agent de neutralisation l'acide arylacétique correspondant à l'arylacétonitrile hydrolysé.

La solution aqueuse neutralisée sortant de (11) est transférée dans une seconde zone de mélange (14) par une conduite (13).

Cette zone de mélange (14) est également de préférence constituée par des mélangeurs statiques tels que mentionnés précédemment et qui peuvent être de type identique ou différent de ceux utilisés dans (1) et (11).

Selon la présente invention, on effectue dans cette zone de mélange (14) une dilution de la solution aqueuse de sel alcalin d'acide arylacétique par de l'eau qui est introduite par (15).

La solution aqueuse ainsi diluée est transférée dans une zone de stockage (17) par la conduite (16).

Selon une variante de l'installation, les moyens de mélange contenus dans l'enceinte (1) peuvent être intégrés dans le réacteur (5).

Dans cette éventualité, les moyens de mélange peuvent être avantageusement disposés à la partie inférieure dudit réacteur (5) ou directement à l'intérieur du ou des tubes, ces moyens de mélange peuvent être constitués par des éléments mélangeurs tels que ceux utilisés dans les mélangeurs statiques tels que mentionnés précédemment.

Le procédé selon la présente invention permet d'obtenir, avec un rendement très élevé en sel alcalin d'acide arylacétique et une transformation quasi quantitative de l'arylacétonitrile correspondant, des solutions aqueuses de sel alcalin d'acide arylacétique pures et exemptes de NH₃. Le procédé présente également l'avantage d'être mis en oeuvre au moyen d'une installation simple, dépourvue notamment de tout système d'agitation du type dynamique qui est de nature à entraîner des fuites et est coûteux en énergie. Le procédé selon la présente invention permet une grande flexibilité. En outre, l'ammoniac formé peut être valorisé sous forme de solutions d'ammoniaque directement commercialisables.

Les solutions aqueuses de sel alcalin d'acide arylacétique peuvent avantageusement être des précurseurs pour obtenir directement des acides arylacétiques par acidification.

Les acides obtenus ne nécessitent pas de moyens de purification coûteux puisque les solutions de départ sont pures.

Les exemples qui suivent illustrent l'invention.

### EXEMPLE 1

On utilise un réacteur tubulaire formé d'un tube en inox d'un diamètre de 2,3 cm et d'une longueur de 18 cm et garni d'anneaux de Raschig laissant un volume utile de 36 ml. Le tube est entouré d'une double enveloppe permettant son chauffage au moyen d'une circulation d'huile.

Le réacteur préalablement rempli avec une solution aqueuse de soude à 26 % et placé sous une pression d'azote de 5 bars est chauffé à 170°C.

On introduit alors 0,25 mol/h (29,5 g/h) de phénylacétonitrile ) préchauffé à 100°C et 0,30 mol/h de NaOH sous forme d'une solution aqueuse à 26 % (46,1 g/h) préchauffée également à 100°C (excès de 20 %) dans le réacteur maintenu à 170°C sous une pression régulée de 8.10⁵ Pa (8 bars).

Le temps de séjour est de 30 minutes.

Le flux sortant du réacteur est récupéré dans une capacité à pression 5 atmosphérique. Après séparation d'une phase transitoire correspondant à la soude initialement présente dans le réacteur, on obtient en régime stabilisé une solution aqueuse de phénylacétate de sodium dont la composition est déterminée par analyse potentiométrique et analyse chromatographique (extraction des organiques par un solvant). La-durée de l'essai en régime stabilisé est de 3h.

Dans ces conditions, la conversion du phénylacétonitrile en phénylacétate de sodium n'est que de 79,5 %.

### EXEMPLE 2

On utilise un réacteur tubulaire formé d'un tube vide en inox d'un diamètre de 2,3 cm et d'une hauteur de 39 cm ayant un volume libre de 162 ml. Le tube est entouré d'une double enveloppe permettant son chauffage au moyen d'une circulation d'huile.

Le réacteur préalablement rempli avec une solution aqueuse de phénylacétate de sodium à 44 % et placé sous une pression d'azote de 3.10⁵ Pa (3 bar) est chauffé à 150°C.

On introduit alors 1,5 mol/h (176g/h) de phénylacétonitrile préchauffé à 100°C et 1,6 mol/h de NaOH sous forme d'une solution aqueuse à 5 % (excès de 6,5 %) dans une enceinte de mélange (par agitation magnétique) connectée à la base du réacteur.

Après un temps de séjour dans ledit mélangeur de 3 minutes, le mélange est introduit dans le réacteur maintenu à 150°C sous une pression régulée de 3.10⁵ Pa (3 bars) le temps de séjour est de 26 minutes.

Le flux sortant du réacteur est récupéré dans une capacité à pression atmosphérique. Après séparation d'une phase transitoire correspondant à la solution de phénylacétate de sodium initialement présente dans le réacteur, on obtient en régime stabilisé une solution aqueuse de phénylacétate de sodium dont la composition est déterminée par analyse potentiométrique et analyse chromatographique (extraction des organiques par un solvant). La durée de l'essai est de 5h en régime stabilisé.

Dans ces conditions, la conversion du phénylacétonitrile en phénylacétate de sodium est de 99,7 %.

### EXEMPLE 3

On utilise le même réacteur tubulaire que dans l'exemple 2 mais garni d'anneaux Raschig laissant un volume utile de 82 ml.

Le réacteur préalablement rempli avec une solution aqueuse de phénylacétate de sodium à 44 % et placé sous une pression d'azote de 5 bars est chauffé à 150°C.

On introduit alors 0,745 mol/h (87,4 g/h) de phénylacétonitrile préchauffé à 110°C et 0,833 mol/h de NaOH sous forme d'une solution aqueuse à 25 % (133,4 g/h) préchauffée à 95°C (excès de 11,8 %) dans la - même enceinte de mélange que dans l'exemple 2.

Après un temps de séjour dans ledit mélangeur de 5,6 minutes, le mélange est introduit dans le réacteur maintenu à 150°C sous une pression régulée d 6.10⁵ Pa (6 bars), le temps de séjour est de 26 minutes.

Le flux sortant du réacteur est récupéré dans une capacité à pression atmosphérique. Après séparation d'une phase transitoire correspondant à la solution de phénylacétate de sodium initialement présente dans le réacteur, on obtient en régime stabilisé une solution aqueuse de phénylacétate de sodium dont la composition est déterminée par analyse potentiométrique et analyse chromatographique (extraction des organiques par un solvant). La durée de l'essai est de 5h en régime stabilisé;

Dans ces conditions, la conversion du phénylacétonitrile en phénylacétate de sodium est de 99,6 %.

### EXEMPLE 4

On utilise un dispositif tel que représenté sur la figure 1.

Dans un mélangeur statique Sulzer (1) en inox 316Ti d'une longueur de 4,8 cm et d'un diamètre de 0,48 cm constitué de 10 éléments de type SMXE DN4 (lamelles croisées) commercialisés par la Société SULZER, on introduit 175 mol/h (20,55 kg/h) de phénylacétonitrile préchauffé à 105°C et 202 mol/h de NaOH (soit un excès molaire de 15,4 %) sous forme d'une solution aqueuse à 25 % (32,33 kg/h) préchauffée également à 105°C.

Après un temps de séjour dans ledit mélangeur de l'ordre de 0,05 seconde, le mélange est introduit dans un réacteur tubulaire en inox 316L constitué de 6 tubes, chaque tube ayant une hauteur égale à 3 m et un diamètre égal à 2,5 cm. Lesdits tubes sont remplis préalablement d'une solution aqueuse de phénylacétate de sodium et sont maintenus à 150°C (chauffage externe avec de la vapeur 12.10⁵ Pa) sous une pression de 6 bars.

Le temps de séjour dans le réacteur est de 8,5 minutes.

Le flux sortant dudit réacteur est introduit par (6) dans un séparateur gaz/liquide (7) constitué d'une capacité en inox 316L de 12 litres.

La solution aqueuse de phénylacétate de sodium sortant du séparateur fortement débarassée de son NH₃ après détente à pression atmosphérique a la composition moyenne suivante :

| | |
|---|---|
| PhCH₂CO₂Na | 55,2 % |
| NaOH | 2,3 % |
| NH₃ | 1,1 % |
| H₂O | 41,4 % |

avec une quantité résiduelle de phénylacétonitrile inférieure à 25 mg/kg.

Cette solution est alors introduite par (8) dans une colonne de stripping (9) de 6 plateaux théoriques constitués par un garnissage ordonné en inox. Le stripping est réalisé à une température de 120°C avec de la vapeur 2.10⁵ Pa (2 bars). tête de colonne (19) sort un flux constitué essentiellement d'ammoniac et d'eau tandis que la solution de phénylacétate de sodium quasi totalement débarassée de son ammoniac (teneur résiduelle inférieure à 50 ppm) est extraite en bas de colonne (9) et est introduite par (10) dans un mélangeur statique (11) de type SMXE.

Dans ce second mélangeur statique (11) on introduit simultanément par (10) la solution provenant de la colonne de stripping et par (12) un courant d'une quantité suffisante d'acide phénylacétique (∼3,7 kg/h) à 90°C pour neutraliser l'excès de soude. La solution sortant du mélangeur statique (11) est ensuite introduite par (13) dans un mélangeur statique (14) identique à (11) en même temps qu'un courant d'eau introduit par (15) afin d'obtenir une solution aqueuse de phénylacétate de sodium ayant une concentration pondérale en phénylacétate de sodium de 44 %.

La durée de l'essai en régime stabilisé est de 4h.

La conversion du phénylacétonitrile est quasi-quantitative et le rendement molaire en phénylacétate de sodium (par rapport au phénylacétonitrile) est supérieur à 99,9 %.

Dans le tableau 1 ci-après, on rapporte les différents paramètres opérationnels ainsi que les taux de transformation du phénylacétonitrile pour l'exemple 4 et aussi les exemples 5, 6 et 7 réalisés selon un processus similaire à celui mis en oeuvre dans l'exemple 4.

**TABLEAU 1**

| **EXEMPLE N°** | **4** | **5** | **6** | **7** |
|---|---|---|---|---|
| Excès molaire de NaOH (en %) | 15,4 | 12,0 | 13,7 | 11,3 |
| Zone de mélange : | | | | |
| - température (en °C) | 105 | 100 | 105,0 | 105 |
| - temps de séjour (en seconde) | 0,05 | 0,05 | 0,07 | 0,05 |
| Zone réactionneile: | | | | |
| - température (en °C) | 150 | 150 | 145 | 135 |
| - temps de séjour (en minutes) | 8,5 | 8,5 | 12 | 8,5 |
| Pression (en Pascal) | 6.10⁵ | 9.10⁵ | 9.10⁵ | 1.10⁶ |
| Concentration moyenne de la solution aqueuse de phénylacétate de sodium en sortie dù séparateur gaz/liquide : | | | | |
| - en C₆H₅CH₂CO₂Na (en %) | 55,2 | 53,5 | 52,6 | 55,3 |
| - en C₆H₅CH₂CN (en ppm) | < 25 | <25 | <50 | <50 |
| Conversion du phénylacétonitrile en phénylacétate de sodium (en %) | >99,9 | >99,9 | >99,9 | >99,9 |
| Durée de l'essai en régime stabilisé (en h) | 4 | 5 | 5,5 | 3 |

### EXEMPLE 8

On utilise le même dispositif que pour l'exemple 4.

Dans le mélangeur statique (1) on introduit 172,1 mol/h (20,2 kg/h) de phénylacétonitrile préchauffé à 107°C et 186,1 mol/h de KOH (soit un excès molaire de 8,1 %) sous forme d'une solution aqueuse à 40,8 % (25,6 kg/h) préchauffée également à 107°C.

Après un temps de séjour dans le mélangeur de l'ordre de 0,065 seconde, le mélange est introduit dans le réacteur tubulaire (5) dont les tubes sont remplis préalablement d'une solution de potasse à 40,8 % et sont maintenus à 150°C sous une pression de 6.10⁵ Pa (6 bars).

Le temps de séjour dans le réacteur est de 10 minutes.

Après une durée de fonctionnement de 30 min permettant d'assurer l'extraction totale de la potasse initialement présente dans le réacteur, on obtient en régime établi en sortie du séparateur gaz/liquide (7) une solution aqueuse de phénylacétate de potassium dont la composition moyenne après détente à la pression atmosphérique est la suivante :

| | |
|---|---|
| PhCH₂CO₂K | 69,6 % |
| KOH | 1,85 % |
| NH₃ | 0,65 % |
| H₂O | 27,9 % |

avec une quantité résiduelle de phénylacétonitrile inférieure à 50 mg/kg.

De façon analogue à l'exemple 4, cette solution subit alors un stripping à la vapeur dans la colonne (9) afin d'obtenir une teneur résiduelle en ammoniac inférieure à 50 mg/kg puis est introduite dans le mélangeur statique (11) avec un courant d'une quantité suffisante d'acide phénylacétique (∼1,9 kg/h) à 90°C pour neutraliser l'excès de potasse. La solution sortant du mélangeur statique (11) est ensuite introduite dans le mélangeur statique (14) en même temps qu'un courant d'eau afin d'obtenir une solution aqueuse de phénylacétate de potassium ayant une concentration pondérale en phénylacétate de potassium de 64 %.

La durée de l'essai en régime stabilisé est de 5h.

La conversion du phénylacétonitrile est quasi-quantitative et le rendement molaire en phénylacétate de potassium (par rapport au phénylacétonitrile) est supérieur à 99,9 %.

## Revendications

1. Procédé continu de préparation d'une solution aqueuse d'un sel alcalin d'acide arylacétique de formule : 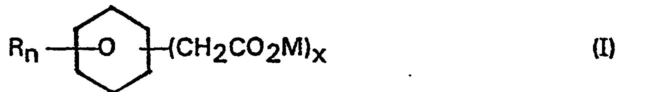 dans laquelle R représente un radical hydrocarboné aliphatique, linéaire ou ramifié ayant un nombre de carbone allant de 1 à 10, un atome d'halogène, un groupe alcoxy linéaire ou ramifié ayant un nombre de carbone allant de 1 à 6, un groupe phénoxy ; M représente un métal alcalin, n est un nombre allant de 0 à 5, et x est un nombre allant de 1 à 2 avec n + x inférieur ou égal à 6 ; par hydrolyse alcaline d'un arylacétonitrile de formule : 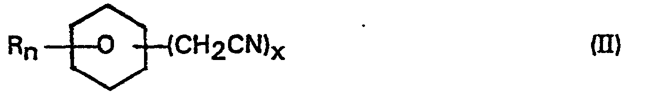 R, n et x ayant les mêmes significations que dans la formule (I) selon la réaction 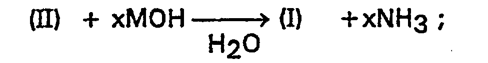 ledit procédé étant **caractérisé en ce qu'**il consiste essentiellement :
a) à mettre en contact dans une zone de mélange un arylacétonitrile (II) avec une solution aqueuse d'un hydroxyde alcalin MOH selon un rapport molaire MOH / (II) au moins égal à x,
b) à introduire le mélange obtenu en a) dans une zone de réaction sous une pression supérieure ou égale à la pression atmosphérique et à maintenir ledit mélange dans ladite zone réactionnelle pendant une durée au plus égale à 60 minutes à une température comprise entre 100°C et 180°C,
c) à transférer le milieu obtenu en b) dans une zone de séparation,
d) à transférer le milieu obtenu en c) dans une zone de stripping,
e) à mettre en contact la solution obtenue en d) avec un agent neutralisant qui est un acide arylacétique de formule 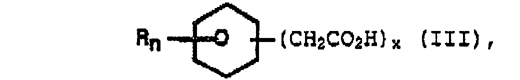 dans une zone de mélange, puis,
f) à récupérer une solution aqueuse d'un sel alcalin d'acide arylacétique pure,

2. Procédé selon la revendication 1, **caractérisé en ce que** le temps de contact entre les réactifs dans la zone de mélange de l'étape a) est, au plus égal à 10 minutes.

3. Procédé selon la revendication 2, **caractérisé en ce que** le temps de contact entre les réactifs dans la zone de mélange de l'étape a) est compris entre 0,01 seconde et 6 minutes.

4. Procédé selon l'une des revendications 1 à 3, **caractérisé en ce que** le mélange obtenu dans l'étape a) est introduit dans une zone de réaction - étape b)- sous une pression comprise entre 2 bars absolus et 12 bars absolus et est maintenu dans ladite zone réactionnelle -pendant une durée comprise entre 5 minutes et 30 minutes à une température comprise entre 130°C et 160°C.

5. Procédé selon l'une des revendications 1 à 4, **caractérisé en ce que** le rapport molaire MOH/(II) est compris entre 1,05x et 1,20x.

6. Procédé selon l'une des revendications 1 à 5, **caractérisé en ce que** la solution aqueuse d'un sel alcalin d'acide arylacétique obtenue dans l'étape e) est dilué avec de l'eau.

7. Procédé selon l'une des revendications 1 à 6, **caractérisé en ce que** n est nul.

8. Procédé selon l'une des revendications 1 à 7, **caractérisé en ce que** x = 1.

9. Procédé selon l'une des revendications 1 à 8, **caractérisé en ce que** le métal alcalin est le sodium ou le potassium.

10. Solution aqueuse de phénylacétate de sodium obtenue selon l'une quelconque des revendications 1 à 9.

11. Solution aqueuse de phénylacétate de potassium obtenue selon l'une quelconque des revendications 1 à 9.

12. Installation comprenant une enceinte (1) contenant des moyens de mélange munie d'une alimentation en arylacétonitrile (2) et d'une alimentation en solution aqueuse d'hydroxyde alcalin (3), une conduite (4) d'admission du mélange dans un réacteur (5) comprenant au moins un tube cylindrique (T) vertical vide, une conduite (6) d'admission de la solution aqueuse contenant un sel alcalin d'acide arylacétique et de l'ammoniac dans une enceinte (7) contenant des moyens de séparation gaz/liquide, une conduite (8) d'admission de la solution aqueuse dégazée dans une colonne de stripping (9), une conduite (10) d'admission de la solution aqueuse strippée dans une enceinte (11) contenant des moyens de mélange munie d'une alimentation en agent neutralisant (12), d'une conduite (13) d'admission dans une enceinte (14) contenant des moyens de mélange munie d'une alimentation (15) en eau, une conduite (16) d'admission de la solution aqueuse du sel alcalin d'acide arylacétique dans une zone de stockage (17) et des évents (18) et (19).

13. Installation selon la revendication 12, **caractérisée en ce que** dans l'enceinte (1), le mélange est réalisé par un mélangeur statique.

14. Installation selon la revendication 13, **caractérisée en ce que** le mélangeur statique comprend au moins un élément mélangeur comprenant des lamelles plissées ou un réseau de lames croisées, emboîtées les unes dans les autres.

15. Installation selon la revendication 12, **caractérisée en ce que** la colonne de stripping (9) contient un garnissage.

16. Installation selon la revendication 12, **caractérisée en ce que** les moyens de mélange contenus dans les enceintes (11) et (14) sont des mélangeurs statiques.

## Patentansprüche

1. Verfahren zur kontinuierlichen Herstellung einer wäßrigen Lösung eines Alkalisalzes einer Arylessigsäure der Formel: 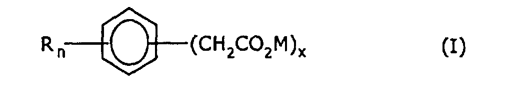 in der R einen gerad- oder verzweigtkettigen aliphatischen Kohlenwasserstoffrest mit 1 bis 10 Kohlenstoffatomen, ein Halogenatom, einen gerad- oder verzweigtkettigen Alkoxyrest mit 1 bis 6 Kohlenstoffatomen, oder eine Phenoxygruppe bedeutet; M ein Alkalimetall, n eine Zahl von 0 bis 5 und x eine Zahl von 1 bis 2 ist, wobei n + x kleiner oder gleich 6 ist; durch alkalische Hydrolyse eines Arylacetonitrils der Formel: 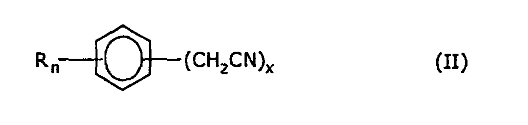 in der R, n und x die gleiche Bedeutung wie in der Formel (I) haben nach der Reaktion 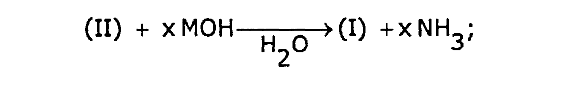 wobei das Verfahren **dadurch gekennzeichnet ist, daß** es im wesentlichen besteht aus:
a) Inkontaktbringen eines Arylacetonitrils (II) mit einer wäßrigen Lösung eines Alkalihydroxids MOH in einem Molverhältnis MOH/(II) wenigstens gleich x in einer Mischzone,
b) Zugabe des bei a) erhaltenen Gemisches in eine Reaktionszone bei einem Druck gleich oder größer dem Atmosphärendruck und Halten des genannten Gemisches in der genannten Reaktionszone für eine Dauer von höchstens 60 Minuten bei einer Temperatur zwischen 100 °C und 180 °C,
c) Überführen des bei b) erhaltenen Milieus in eine Trennzone,
d) Überführen des bei c) erhaltenen Milieus in eine Abstreifzone,
e) Inberührungbringen der bei d) erhaltenen Lösung mit einer Arylessigsäure der Formel 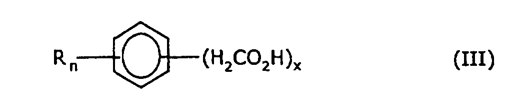 als Neutralisationsmittel in einer Mischzone und dann
f) Gewinnung in einer reinen wäßrigen Lösung eines Alkalisalzes von Arylessigsäure.

2. Verfahren nach Anspruch 1, **dadurch gekennzeichnet, daß** die Berührungszeit zwischen den Reagenzien in der Mischzone der Stufe a) höchstens 10 Minuten beträgt.

3. Verfahren nach Anspruch 2, **dadurch gekennzeichnet, daß** die Berührungszeit zwischen den Reagenzien in der Mischzone der Stufe a) zwischen 0,01 Sekunden und 6 Minuten liegt.

4. Verfahren nach einem der Ansprüche 1 bis 3, **dadurch gekennzeichnet, daß** das in der Stufe a) erhaltene Gemisch der Reaktionszone - Stufe b) - unter einem Druck zwischen 2 und 12 bar absolut zugeführt und in dieser Reaktionszone für eine Dauer zwischen 5 Minuten und 30 Minuten bei einer Temperatur zwischen 130 °C und 160 °C gehalten wird.

5. Verfahren nach einem der Ansprüche 1 bis 4, **dadurch gekennzeichnet, daß** das Molverhältnis MOH/(II) zwischen 1,05 x und 1,20 x liegt.

6. Verfahren nach einem der Ansprüche 1 bis 5, **dadurch gekennzeichnet, daß** die in der Stufe e) erhaltene wäßrige Lösung eines Alkalisalzes der Arylessigsäure mit Wasser verdünnt wird.

7. Verfahren nach einem der Ansprüche 1 bis 6, **dadurch gekennzeichnet, daß** n Null ist.

8. Verfahren nach einem der Ansprüche 1 bis 7, **dadurch gekennzeichnet, daß** x = 1 ist.

9. Verfahren nach einem der Ansprüche 1 bis 8, **dadurch gekennzeichnet, daß** das Alkalimetall Natrium oder Kalium ist.

10. Wäßrige Lösung von Natriumphenylacetat, erhalten nach einem der Ansprüche 1 bis 9.

11. Wäßrige Lösung von Kaliumphenylacetat, erhalten nach einem der Ansprüche 1 bis 9.

12. Anlage enthaltend einen Behälter (1) mit Mischeinrichtungen, der mit einer Zufuhr für Arylacetonitril (2) und einer Zufuhr für die wäßrige Lösung eines Alkalihydroxids (3) und einer Leitung (4) zur Überführung in einen Reaktor (5) versehen ist, der wenigstens ein senkrechtes leeres zylindrisches Rohr (T), eine Leitung (6) für die Zufuhr der wäßrigen, ein Alkalisalz der Arylessigsäure enthaltenen Lösung und von Ammoniak in einen Behälter (7) vorsieht, mit Mitteln zur Trennung von Gas/Flüssigkeit und einer Leitung (8) für die Zufuhr der entgasten wäßrigen Lösung in eine Abstreifkolonne (9) enthält, mit einer Leitung (10) für die Zufuhr der abgestreiften wäßrigen Lösung in einen Raum (11) mit Mischeinrichtungen, der mit einer Zufuhr für das Neutralisationsmittel (12) und einer Leitung (13) für die Zufuhr in den mit Mischeinrichtungen ausgerüsteten Behälter (14) versehen ist, der eine Wasserleitung (15) und eine Leitung (16) für die Abgabe der wäßrigen Lösung des Alkalisalzes der Arylessigsäure in eine Aufbewahrungszone und Belüftungen (18) und (19) aufweist.

13. Anlage nach Anspruch 12, **dadurch gekennzeichnet, daß** in dem Behälter (1) die Mischung mit einem statischen Mischer verwirklicht wird.

14. Anlage nach Anspruch 13, **dadurch gekennzeichnet, daß** der statische Mischer wenigstens ein Mischelement mit gefalteten Lamellen oder ein Gitter von gekreuzten ineinandergreifenden Klingen enthält.

15. Anlage nach Anspruch 12, **dadurch gekennzeichnet, daß** die Abstreifkolonne (9) eine Füllkörperschüttung enthält.

16. Anlage nach Anspruch 12, **dadurch gekennzeichnet, daß** die in den Behältern (11) und (14) angeordneten Mischvorrichtungen statische Mischer sind.

## Claims

1. Continuous process for the preparation of an aqueous solution of an alkaline salt of arylacetic acid of formula: 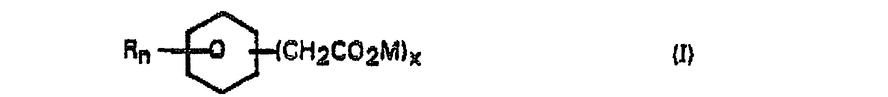 in which R represents a linear or branched aliphatic hydrocarbon radical having a carbon number ranging from 1 to 10, a halogen atom, a linear or branched alkoxy group having a carbon number ranging from 1 to 6, or a phenoxy group; M represents an alkali metal, n is a number ranging from 0 to 5, and x is a number ranging from 1 to 2 with n + x less than or equal to 6 ; by alkaline hydrolysis of an arylacetonitrile of formula : 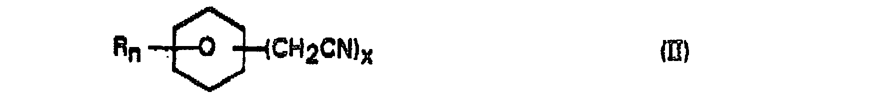 R, n and x having the same meanings as in formula (I), according to the reaction 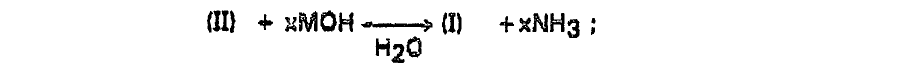 the said process being **characterized in that** it consists essentially:
a) in placing in contact, in a mixing zone, an arylacetonitrile (II) with an aqueous solution of an alkaline hydroxide MOH in an MON/(II) molar ratio at least equal to x,
b) in introducing the mixture obtained in a) into a reaction zone under a pressure greater than or equal to atmospheric pressure and in maintaining the said mixture in the said reaction zone for a period at most equal to 60 minutes at a temperature of between 100°C and 180°C,
c) in transferring the medium obtained in b) into a separation zone,
d) in transferring the medium obtained in c) into a stripping zone,
e) in placing the solution obtained in d) in contact with a neutralizing agent, which is an arylacetic acid of formula 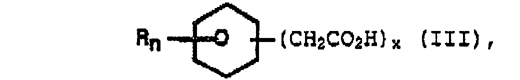 in a mixing zone, and then,
f) in recovering a pure aqueous solution of an alkaline salt of arylacetic acid.

2. Process according to Claim 1, **characterized in that** the contact time between the reactants in the mixing zone of step a) is at most equal to 10 minutes.

3. Process according to Claim 2, **characterized in that** the contact time between the reactants in the mixing zone of step a) is between 0.01 second and 6 minutes.

4. Process according to one of Claims 1 to 3, **characterized in that** the mixture obtained in step
a) is introduced into a reaction zone = step b) - under a pressure of between 2 bar absolute and 12 bar absolute, and is maintained in the said reaction zone for a period of between 5 minutes and 30 minutes at a temperature of between 130°C and 160°C.

5. Process according to one of claims 1 to 4, **characterized in that** the MOH/(II) molar ratio is between 1.05x and 1.20x.

6. Process according to one of Claims 1 to 5, **characterized in that** the aqueous solution of an alkaline salt of arylacetic acid, obtained in step e), is diluted with water.

7. Process according to one of claims 1 to 6, **characterized in that** n is zero.

8. Process according to one of Claims 1 to 7, **characterized in that** x = 1.

9. Process according to one of Claims 1 to 8, **characterized in that** the alkali metal is sodium or potassium.

10. Aqueous sodium phenylacetate solution obtained according to any one of Claims 1 to 9.

11. Aqueous potassium phenylacetate solution obtained according to any one of Claims 1 to 9.

12. Plant comprising a chamber (1) containing mixing means, furnished with a supply of arylacetonitrile (2) and a supply of aqueous solution of alkaline hydroxide (3), a pipe (4) for introducing the mixture into a reactor (5) comprising at least one vertical cylindrical drain tube (T), a pipe (6) for introducing the aqueous solution containing an alkaline salt of arylacetic acid and ammonia into a chamber (7) containing gas/liquid separation means, a pipe (8) for introducing the degassed aqueous solution into a stripping column (9), a pipe (10) for introducing the stripped aqueous solution into a chamber (11) containing mixing means, furnished with a supply of neutralizing agent (12), a pipe (13) for introducing [lacuna] into a chamber (14) containing mixing means, furnished with a supply (15) of water, a pipe (16) for introducing the aqueous solution of the alkaline salt of arylacetic acid into a storage zone (17), and vent pipes (18) and (19).

13. Plant according to Claim 12, **characterized in that** in the chamber (1), the mixing is carried out by a static mixer.

14. Plant according to Claim 13, **characterized in that** the static mixer comprises at least one mixing element comprising pleated lamellae or a network of crossed, interlocking blades.

15. Plant according to Claim 12, **characterized in that** the stripping column (9) contains packing.

16. Plant according to Claim 12, **characterized in that** the mixing means contained in the chambers (11) and (14) are static mixers.
